# EUROPÄISCHE PATENTSCHRIFT

(11) **EP 0 692 987 B1**
(45) Veröffentlichungstag und Bekanntmachung des Hinweises auf die Patenterteilung: **15.10.1997**
(21) Anmeldenummer: 94909105.2
(22) Anmeldetag: 02.03.1994
(51) Int. Cl.: A61M 1/00

(54) **PLEURADRAINAGEVORRICHTUNG**
PLEURAL-CAVITY DRAINAGE DEVICE
DISPOSITIF DE DRAINAGE PLEURAL

(30) Priorität: 02.03.1993 DE 4306478
(43) Veröffentlichungstag der Anmeldung: 24.01.1996
(73) Patentinhaber: Wagner, Wolfgang, Dr., 22765 Hamburg (DE)
(72) Erfinder: Wagner, Wolfgang, Dr., 22765 Hamburg (DE)
(74) Vertreter: VOSSIUS & PARTNER
(86) Internationale Anmeldenummer: EP9400613
(87) Internationale Veröffentlichungsnummer: WO9420152

(56) Entgegenhaltungen:
- US-A- 3 066 672
- US-A- 4 592 741
- US-A- 4 664 660
- US-A- 4 735 606
- US-A- 5 029 580

## Beschreibung

Bei der herkömmlichen Drainage bzw. dem Absaugen von Fluiden aus der Pleurahöhle, wird üblicherweise ein einlumiger Schlauch verwendet, der mittels eines Trocars oder operativ in die Körperhöhle verbracht wird. Durch Anschluß des Schlauchs an eine Unterdruckquelle wird Fluid, wie Luft oder Flüssigkeit, aus der Pleurahöhle abgesaugt.

Die meisten herkömmlichen Drainagesysteme für Körperhöhlen erlauben keine Messung der Strömungsgeschwindigkeit. Oft ist nicht einmal eine Anzeige vorhanden, ob überhaupt eine Strömung aus der Körperhöhle vorliegt. Es sind allerdings auch Konstruktionen bekannt, bei welchen die Strömung angezeigt wird, zum Beispiel dadurch, daß der abgesaugte Gasstrom durch eine Flüssigkeit geführt wird, wobei das Vorliegen einer Strömung durch aufsteigende Blasen angezeigt wird.

Andererseits ist eine quantitative Angabe sowohl über die initiale als auch über die eventuell fortlaufend abgesaugte Gas- oder Flüssigkeitsmenge eine klinisch wichtige Information. So kann beispielsweise bei einer fortbestehenden Leckage zwischen dem Bronchialsystem und dem Pleuraraum aus der Menge des pro Minute in die Pleurahöhle nachströmenden Atemgases beurteilt werden, ob ein spontaner Verschluß der Leckage zu erwarten ist, oder ob ein operatives Vorgehen erwogen werden sollte.

Eine exakte Messung der Menge abgesaugter Fluide wäre auch deshalb wünschenswert, weil der optimale Unterdruck im Drainagesystem nur per Durchflußmessung bestimmt werden kann. Optimal ist der Unterdruck bzw. Sog dann, wenn eine maximale Gas- oder Flüssigkeitsmenge pro Zeitintervall abgesaugt wird. Dies ist nicht notwendigerweise ein möglichst hoher Sog, denn ein zu hoher Sog führt oft zu einem Haften von anatomischen Strukturen, z.B. peripheren Lungenanteilen bei der Pleuradrainage, oder Gerinnseln an den Saugöffnungen des Schlauchs und somit zu einem Verschluß der Drainage.

Die herkömmlichen Drainagesysteme sind auch störanfällig. So vermindert beispielsweise Flüssigkeit, die in durchhängenden Teilen des Schlauches liegt, den patientenseitigen Unterdruck oder hebt ihn ganz auf. Dasselbe gilt für im Schlauch geronnene Flüssigkeit und für Knickstellen. Derartige Störungen treten häufig auch im nichteinsehbaren Teil des Drainagesystems von der Verbandabdeckung bis zur Körperhöhle, beispielsweise zum Thoraxinnenraum, auf und sind deshalb nur schwer erkennbar. Es kann sich dann immer noch oder gegebenenfalls erneut Gas oder Flüssigkeit in der Körperhöhle des Patienten ansammeln. Einen Hinweis darauf können nur gründliche Untersuchungsverfahren, wie Auskultation (Abhören) oder Röntgen, geben. Wenn derartige Probleme nicht rechtzeitig erkannt werden, können auch kritische Symptome seitens des Patienten auftreten.

Aus der DE-C2-34 30 095 ist eine Vorrichtung zur Spülung von Körperhohlorganen sowie deren Verwendung zur antikonzeptiven Gebärmutter-Spülung und zur enteralen/intestinalen Dialyse bekannt, mit der in einem sonst schwer oder nicht erreichbaren Hohlraum intensiv und gleichzeitig schonend Spülungen zur Behandlung von Erkrankungen vorgenommen werden sollen. Hierbei ist zur Zuführung der Spülflüssigkeit ein Druckschlauch vorgesehen, der an seinem vorderen Ende einen Düsenkopf mit nach hinten gerichteten Düsen und außerdem in seinem vorderen Bereich zusätzlich mehrere nach hinten gerichtete Düsen für den Vortrieb im Körper und für die Spülung aufweist. Ein Absaugschlauch weist in seinem vorderen Bereich ebenfalls seitlich Durchbrechungen auf. Somit findet im gesamten vorderen Bereich der Spülvorrichtung eine Fluidkommunikation zwischen Druckschlauch und Absaugschlauch statt, und der Druckschlauch und der Absaugschlauch werden nicht in ihrer ganzen Länge axial durchspült. Eine Überprüfung und gegebenenfalls Wiederherstellung der Durchgängigkeit der Spülvorrichtung wird nicht angestrebt.

Aus der US-A-4 650 462 ist ein Durchspülungssystem zur Verwendung bei endoskopischen Verfahren, insbesondere bei der arthroskopischen Chirurgie bekannt. Das Durchspülungssystem weist eine Zuführleitung, eine Leitung zum Messen und Steuern des Drucks und eine abführende Leitung auf; hierdurch sollen Druck und Strömung der Spülflüssigkeit unabhängig voneinander gemessen werden können. Die Zuführleitung, die Drucksteuerleitung und die abführende Leitung sind voneinander getrennt angeordnet.

Aus der US-A-4 735 606 ist eine Brustdrainage und insbesondere eine Mediastinaldrainage bekannt, der die Aufgabe zugrunde liegt, das Zusetzen des Drains und das verdunstungsbedingte Absinken des Flüssigkeitsspiegel in einer Blubberkontrolle zu verhindern. Zu diesem Zweck weist die Drainagevorrichtung ein mit einer Vakuumquelle verbundenes Hauptlumen auf, wobei das abgesaugte Gas durch die Blubberkontrolle fließt, sowie ein mit dem Hauptlumen in Fluidverbindung stehendes Hilfslumen. In der Zufuhrleitung zu dem Hilfslumen ist ein einstellbares Ventil vorgesehen, durch das der Strömungswiderstand der Atmosphärenluft zu dem Hilfslumen variiert werden kann, um so das Blubbern des Gases in der Blubberkontrolle einzustellen, wenn die Drainage in Betrieb ist. Hauptlumen und Hilfslumen können zumindest teilweise als doppelläufiger Schlauch ausgebildet sein.

Der Erfindung liegt somit die Aufgabe zugrunde, eine Drainagevorrichtung und ein Verfahren zum Absaugen von Fluiden aus einer Pleurahöhle bereitzustellen, mit denen die vorstehenden Probleme vermieden werden können und ein störungsfreier Verlauf der Drainage sichergestellt wird, insbesondere die Durchgängigkeit des Drainagesystems einfach sichergestellt und überwacht werden kann.

Diese Aufgabe wird durch die Merkmale der Patentansprüche gelöst. Die Erfindung geht dabei von dem Grundgedanken aus, einen doppelläufigen Schlauch bzw. eine doppelläufige Sonde bereitzustellen, wobei ein Lumen des Schlauchs dem Absaugen von Fluiden aus einer Pleurahöhle und ein weiteres Lumen des Schlauchs der permanenten oder intermittierenden Zufuhr eines Gases in die Pleurahöhle dient, so daß praktisch eine Durchspülung der Körperhöhle stattfinden kann. Üblicherweise ist das zuführende Lumen der Sonde kleiner als das abführende Lumen. Das zuführende Lumen bzw. die Zuführleitung ist durchgehend ausgebildet in dem Sinne, daß ihre Wandung keine Durchbrechnungen aufweist und von ihrem distalen Ende bis zum patientenseitigen Ende, wo das Lumen in Fluidverbindung mit dem abführenden Lumen steht, geschlossen ist. Das abführende Lumen bzw. der Drainageschlauch weist lediglich in der Nähe seines patientenseitigen Endes Öffnungen oder Durchbrechungen zur Aufnahme der Fluide aus der Körperhöhle auf. Gegebenenfalls kann auch der Drainageschlauch durchgehend sein und das zuführende Lumen Öffnungen oder Durchbrechungen in der Nähe seines patientenseitigen Endes aufweisen.

Die Drainagevorrichtung basiert somit auf dem Prinzip einer möglichst vollständigen axialen Durchströmung von Zuführleitung und Absaugschlauch bei gleichzeitiger Vermeidung retrograder Wiedereinspülung von möglicherweise infektiösem Material über den Absaugschlauch.

Erfindungsgemäß ist dabei eine Einrichtung zum Bestimmen der Differenz der Strömungen in den beiden Lumen der Sonde vorgesehen.

Neben einem zum Unterdrucksystem führenden Drainageschlauch ist eine zweite Leitung bzw. Zusatzleitung vorgesehen, die am patientenseitigen Ende, wo sich die Absaugöffnung des Drainageschlauchs befindet, mit dem Lumen des Drainageschlauchs verbunden ist. Vorzugsweise weist hierzu der Drainageschlauch selbst ein zweites Lumen bzw. eine zweite Lichtung auf, die die Zusatzleitung bildet. Die Zusatzleitung kann im Vergleich zum Drainageschlauch einen relativ kleinen Querschnitt aufweisen. Vorzugsweise ist die Zusatzleitung über einen Bakterienfilter, ein Druckmeßgerät, einen Durchflußmesser und ein Sicherheitssperrventil mit der Außenluft verbunden. Außerdem kann, vorzugsweise patientenseitig vom Bakterienfilter, ein Zuspritzventil angeordnet sein, über das beispielsweise Flüssigkeiten zum gelegentlichen Freispülen oder Medikamente, wie Lokalanästhetika oder Antibiotika, in das System eingebracht werden können.

Der Drainageschlauch, der den absaugenden Schenkel der Drainagevorrichtung bildet, führt in der Regel, wie bisher üblich, in ein Abscheidegefäß. Das Abscheidegefäß ist über einen weiteren Durchflußmesser mit einer Unterdruckquelle verbunden. Die Unterdruckquelle kann in bekannter Weise Einrichtungen zur Unterdruckmessung, zur Vermeidung einer Strömungsumkehr und/oder Störungsalarmeinrichtungen, aufweisen.

Im Betrieb der erfindungsgemäßen Pleuradrainagevorrichtung strömt einerseits ein Gas, vorzugsweise Luft, über die Zusatzleitung in die Pleurahöhle, während andererseits die abgesaugten Fluide aus der Pleurahöhle ausströmen. Das erfindungsgemäße Meßprinzip besteht darin, die Differenz zwischen dem Zufluß und dem Abfluß der Pleurahöhle zu messen. Vorzugsweise wird diese Differenz über ein bestimmtes Zeitintervall integriert. Aus dieser Differenz läßt sich dann einerseits die Höhe der geförderten Fluidmenge zu Beginn der Drainage und andererseits die Menge des bei weiterbestehender Leckage kontinuierlich oder phasisch in die Pleurahöhle austretenden Atemgases ermitteln. Gegebenenfalls kann zur Bestimmung des Unterschieds zwischen den Strömungen auch nur die Strömung im abführenden Schenkel gemessen werden. Es entsteht dann eine Differenz zwischen dem Fluß, der gemessen wird, wenn das System geöffnet ist, und dem Fluß in geschlossenem Zustand. Auf diese Weise ist der Durchflußmesser im zuführenden Schenkel entbehrlich.

Das Vorliegen einer Strömung in beiden Leitungen, d.h. der zuführenden Zusatzleitung und dem abführenden Drainageschlauch, zeigt die Durchgängigkeit an. Diese Strömung, die auch ohne patientenseitigen Beitrag besteht, sorgt für eine ständige Entleerung anfallender Flüssigkeiten aus dem Schlauchsystem in das Abscheidegefäß.

Die erfindungsgemäße Pleuradrainagevorrichtung kann kontinuierlich betrieben werden. Vorzugsweise kann die Drainagevorrichtung aber auch so betrieben werden, daß die Zusatzleitung bzw. der zuführende Schenkel intermittierend geöffnet oder wahlweise zugeschaltet wird. Ist die Zusatzleitung gesperrt, reduziert sich die Differenz der beiden Durchflüsse auf die Strömung im abführenden Teil (Drainageschlauch), die dann unmittelbar das vom Patienten kommende Fluidvolumen pro Zeiteinheit ergibt. Das intermittierende Öffnen der Zusatzleitung dient einerseits der Durchspülung, d.h. dem Absaugen von Flüssigkeit, und andererseits der Kontrolle, ob das Drainagesystem weiterhin durchgängig ist. Beim intermittierenden Betrieb wird beim Schließen der Zusatzleitung zunächst Flüssigkeit eingesaugt, die dann beim Öffnen abgesaugt bzw. abtransportiert wird, da kein Sogausgleich stattfindet. Die in der Öffnungsphase herrschende Strömung zeigt die Durchgängigkeit des Systems an. Aus der Differenz zwischen Zu- und Abstrom wird die geförderte Gas- und Flüssigkeitsmenge bestimmt. Dabei ist die initiale Fördermenge bekannt, und auch diejenige Fördermenge, die bei persistierender Leckage weiterhin anfällt. Außerdem kann beim intermittierenden Betrieb die Gefahr verringert werden, daß es beim Ansaugen von unbefeuchteter Luft zu einer Austrocknung von anatomischen Strukturen kommt, die in der Nähe der Absaugöffnungen, d.h. am pleuralen Ende der Sonde liegen.

Bei der erfindungsgemäßen Pleuradrainagevorrichtung ist vorzugsweise vorgesehen, daß im Fall einer negativen Differenz zwischen Abfuhr und Zufuhr, d.h., daß in dem zuführenden Schenkel der Zusatzleitung eine höhere Strömung herrscht als im abführenden Schenkel des Drainageschlauchs, die Zusatzleitung automatisch gesperrt wird, um eine Gasvermehrung im Pleuraraum zu verhindern.

Die wahlweise Zuschaltung der Zusatzleitung ist beispielsweise dann vorteilhaft, wenn bei zunächst unkompliziertem Krankheitsverlauf keine aufwendigen Fluidströmungsbestimmungen erforderlich sind. Vorzugsweise ist dabei zwischen dem Zuspritzventil und dem Bakterienfilter ein Druckmeßgerät in die Zusatzleitung geschaltet, z.B. ein Unterdruckmanometer, und stromaufwärts vom Bakterienfilter, d.h. im patientenfernen Teil der Zusatzleitung, ist ein Belüftungsventil vorgesehen.

Im Betrieb der Drainagevorrichtung bei geschlossener Zusatzleitung zeigt das Manometer bei fehlendem Gas- bzw. Flüssigkeitszufluß aus dem Thoraxraum einen Unterdruck an, der mit dem vor der Drainage anliegenden Unterdruck identisch ist. Bei Zufluß aus dem Thoraxraum zeigt das Manometer dagegen den Druck in der Pleurahöhle an, also einen geringeren Druck. Die Druckdifferenz zeigt somit eine Strömung aus dem Pleuraraum in die Saugung an, und bei bekanntem Leitungswiderstand kann daraus dann das Strömungsvolumen pro Zeitintervall bestimmt werden. Ein bekannter Leitungswiderstand ist dann anzunehmen, wenn eine Einengung oder Verlegung des Systems ausgeschlossen werden kann.

Beim Betätigen (Öffnen) des Belüftungsventils in der Zusatzleitung wird der Unterdruck im ansaugenden Schenkel (Zusatzleitung) abgebaut und die im abführenden Schenkel (Drainageschlauch) liegende Flüssigkeit wird in das Abscheidegefäß entleert. Wird danach das Belüftungsventil wieder geschlossen, erreicht der durch das Manometer angezeigte Unterdruck im ansaugenden Schenkel im Normalbetrieb wieder den vorherigen Wert. Ein Verbleiben der Anzeigen des Manometers auf dem Wert, der bei geöffnetem Belüftungsventil angezeigt wurde, oder eine verlangsamte Druckverringerung zeigt hingegen eine teilweise oder vollständige Verlegung des Systems an. In diesem Fall kann dann versucht werden, durch Zuspritzen von steriler Spülflüssigkeit, beispielsweise physiologischer NaCl-Lösung, und mehrmaliges Betätigen des Belüftungsventils das System wieder durchgängig zu machen. Hingegen ist bei der herkömmlichen Pleuradrainage mit einlumiger Sonde im Falle einer derartigen Störung regelmäßig das Anlegen einer neuen Drainagesonde, d.h. ein neuerlicher operativer Eingriff, erforderlich.

Da das Manometer in der Zusatzleitung in geschlossenem Zustand der Zusatzleitung den Pleuraldruck anzeigt, gestattet auch diese Ausführungsform mit zuschaltbarer Zusatzleitung die Ermittlung eines hinreichenden, aber nicht zu hohen Absaugunterdrucks. Ein positiver Pleuradruck als Ausdruck eines Pneumothorax muß sich bei ausreichendem Sog in Richtung Null bewegen. Ein Festschlagen der Saugöffnung würde sich durch einen Sprung des Manometers auf den vollen Betrag des Absaugunterdrucks bemerkbar machen; ein bei probatorisch abgeklemmter abführender Leitung (Drainageschlauch) positiver Pleuraldruck würde nach Abnahme der Klemme auf einen negativen Wert abfallen.

Die erfindungsgemäße Drainagevorrichtung erlaubt eine rasche Störungserkennung und -beseitigung und die Ermittlung des optimalen Sogs. Insbesondere bei kompliziertem Verlauf mit persistierender bronchopleuraler Leckage kann mit der Durchflußmessung und gegebenenfalls einer automatischen Verschlußphasensteuerung die drainierte Fluidmenge bestimmt werden, d.h. durch Kenntnis von Ausmaß und Verlauf der Leckage wird die Indikationsstellung zu radikaleren therapeutischen Maßnahmen (z.B. Thorakotomie) für den behandelnden Arzt erleichtert, und es wird die Optimierung der maschinellen Beatmung als Kompromiß zwischen atemmechanischen Erfordernissen einerseits und Niedrighaltung des Leckagevolumens andererseits ermöglicht.

Ferner kann in der erfindungsgemäßen Pleuradrainagevorrichtung eine Einrichtung zum Auftrennen der Vorrichtung vorgesehen sein, beispielsweise in Form eines Kopplungsstückes, um einen Manipulatordraht einzuführen. Vorzugsweise ist dieses Kopplungsstück in der Zuführleitung angeordnet, um zu verhindern, daß durch den Manipulatordraht eine retrograde Wiedereinspülung von möglicherweise infektiösem Material erfolgen kann. Der Manipulatordraht besteht vorzugsweise aus einem leicht biegbaren Material und dient dazu, die Lage der Spitze der Drainagevorrichtung, d.h. der Sonde in der Körperhöhle des Patienten abzuändern, um eine optimale Drainage zu erzielen.

Die Erfindung wird nachstehend anhand der Zeichnungen näher erläutert. Es zeigen:
- Fig. 1: das patientenseitige Ende einer in einer erfindungsgemäßen Pleuradrainagevorrichtung verwendeten doppelläufigen Drainagesonde,
- Fig. 2a,b: einen Querschnitt der Drainagesonde sowie einen Mandrin zur Einbringung der Sonde in den Pleuraraum,
- Fig. 3: eine vergrößerte Darstellung der Drainagesonde,
- Fig. 4: eine Ausführungsform einer erfindungsgemäßen Pleuradrainagevorrichtung,
- Fig. 5: eine weitere Ausführungsform einer Pleurarainagevorrichtung mit zuschaltbarer Zusatzleitung,
- Fig. 6: einen Manipulatordraht zum Verändern der Lage der Drainagesonde,
- Fig. 7: eine herkömmliche Pleuradrainage (Thoraxdrainage) nach Bülau, und
- Fig. 8: verschiedene Störungsmöglichkeiten bei der herkömmlichen Pleuradrainage gemäß Figur 7.

Bei der Darstellung der herkömmlichen Pleuradrainage nach Bülau in Figur 7 ist mit 10 die Lunge des Patienten, mit 12 die Pleurahöhle (Pleuraraum) und mit 13 das Rippenfell (Pleura parietalis) bezeichnet. Der Drainageschlauch (Drain) 14 ist mit seiner Ansaugöffnung 16 in die Pleurahöhle 12 des Patienten eingelegt. Die Pfeile im Drainageschlauch 14 geben die Strömungsrichtung an. Ein Flüssigkeitsabscheidegefäß 18 nimmt Ergußflüssigkeit, Blut etc. auf. Eine Durchflußmeßvorrichtung (Flowkontrolle) 20 zeigt das Vorhandensein einer Strömung, d.h. den aktuellen Abtransport eines Fluides, wie Gas oder Flüssigkeit, aus der Pleurahöhle 12 des Patienten an, wenn in einer in der Durchflußmeßvorrichtung enthaltenden Flüssigkeit 21 Blasen aufsteigen. Ein Wasserschloß 22 dient als Druckregelung und begrenzt durch den Einstrom von atmosphärischer Luft durch das mittlere Rohr 23 des Wasserschlosses 22 den Unterdruck auf die durch die Eintauchtiefe des mittleren Rohrs 23 gegebene Höhe. Der Unterdruck wird durch eine Saugpumpe 24 erzeugt und an einem Manometer 26 gemessen. Die drei Einheiten 18, 20 und 22 sowie das Manometer 26 können auch in einer einzigen Überwachungsvorrichtung zusammengefaßt sein.

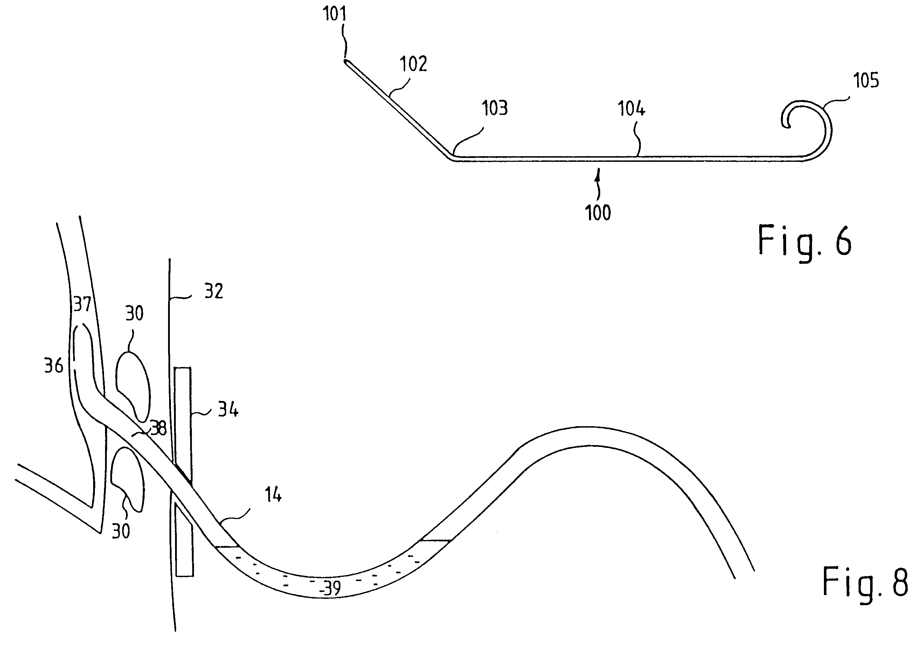

Figur 8 zeigt verschiedene Störungsmöglichkeiten an einer herkömmlichen Pleuradrainage gemäß Figur 7. Dabei sind die Rippen des Patienten mit 30, die Haut mit 32 und ein Verband mit 34 bezeichnet. Bei 36 und 37 kann eine Verlegung der Drainage durch anliegende Lunge oder Rippenfell oder durch geronnene Flüssigkeit im Pleuraspalt auftreten. Bei 38 kann eine Verlegung durch Abknickung oder geronnene Flüssigkeit auftreten. Bei 39 kann schließlich eine Verlegung durch Flüssigkeit in einem durchhängenden Schlauchabschnitt auftreten. Dabei ist zu beachten, daß die Grenze der optischen Kontrollmöglichkeit durch den Verband 34 gegeben ist, so daß beispielsweise die Verlegungen bei 36, 37 und 38 durch optische Kontrolle nicht erkannt werden können. Allerdings werden in der Praxis auch Verlegungen durch Flüssigkeiten in durchhängenden Schlauchabschnitten, wie bei 39, nicht immer rechtzeitig erkannt.

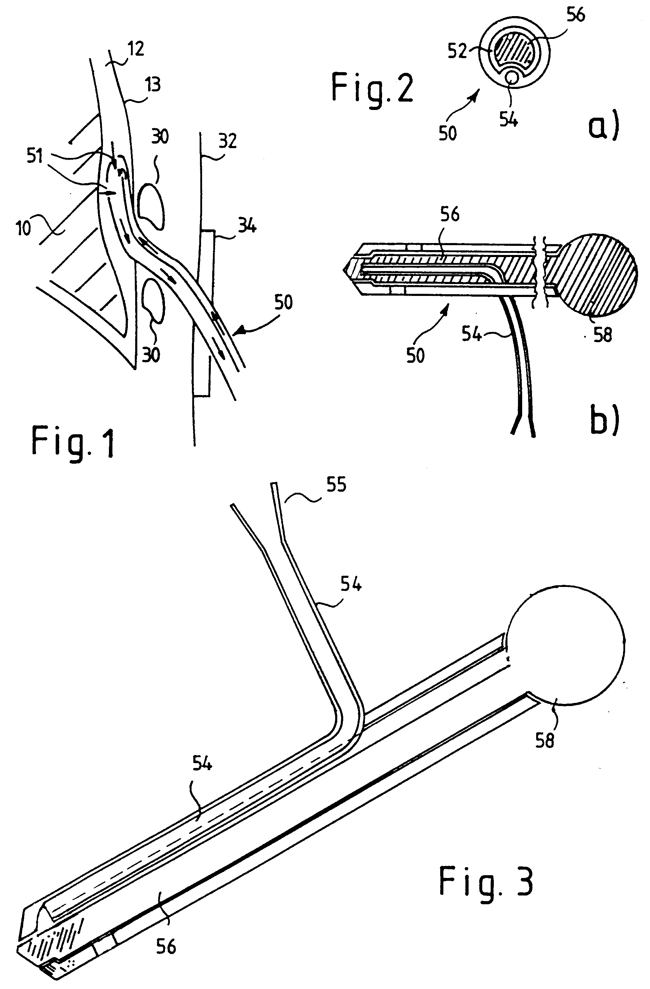

Figur 1 zeigt das patientenseitige Ende eines in der erfindungsgemäßen Pleuradrainagevorrichtung verwendeten doppelläufigen Drains 50 mit zwei Ansaugöffnungen 51 in der Nähe des patientenseitigen Endes der Drainagevorrichtung. Dabei sind in Figur 1 gleiche Teile mit den gleichen Bezugszeichen wie in den Figuren 7 und 8 bezeichnet.

Figuren 2a und 2b zeigen eine Möglichkeit der Einbringung der Drainagesonde 50. Dabei ist mit 52 der abführende Teil der Sonde bezeichnet, der in seiner Funktion einem herkömmlichen Drainageschlauch entspricht. Mit 54 ist das zuführende Lumen bzw. die zuführende Lichtung bezeichnet. Der Unterschied in der Lichtungsweite zwischen zuführendem und abführendem Teil sorgt dafür, daß der Unterdruck am pleuranahen Sondenende etwa dem am patientenfernen Ende mittels einer Unterdruckquelle applizierten Sog gleicht und stellt den größeren Querschnittsanteil dem materialabführenden Teil der Sonde zur Verfügung. Sowohl der Querschnitt gemäß Figur 2a als auch die geschnittene Seitenansicht gemäß Figur 2b zeigt einen stabilen Mandrin 56, mit dem die Sonde in den Pleuraraum des Patienten eingebracht werden kann und der in das größere abführende Lumen 52 der Sonde 50 eingelegt wird. Mit 58 ist ein Griff des Mandrins 56 bezeichnet.

In der vergrößerten schematischen Skizze gemäß Figur 3 ist am Ende der zuführenden Leitung (Zusatzleitung) 54 ein Anschluß 55 zur Ankopplung desjenigen Teils der Zusatzleitung erkennbar, der ein Zuspritzventil, einen Bakterienfilter, eine Druckmeßvorrichtung, ein Sperrventil und ein Durchflußmeßgerät aufnehmen kann. Die abführende Leitung kann ebenfalls einen Anschluß aufweisen, an dem ein weiterer Schlauchteil angekoppelt wird, der zum Flüssigkeitsabscheidegefäß führt.

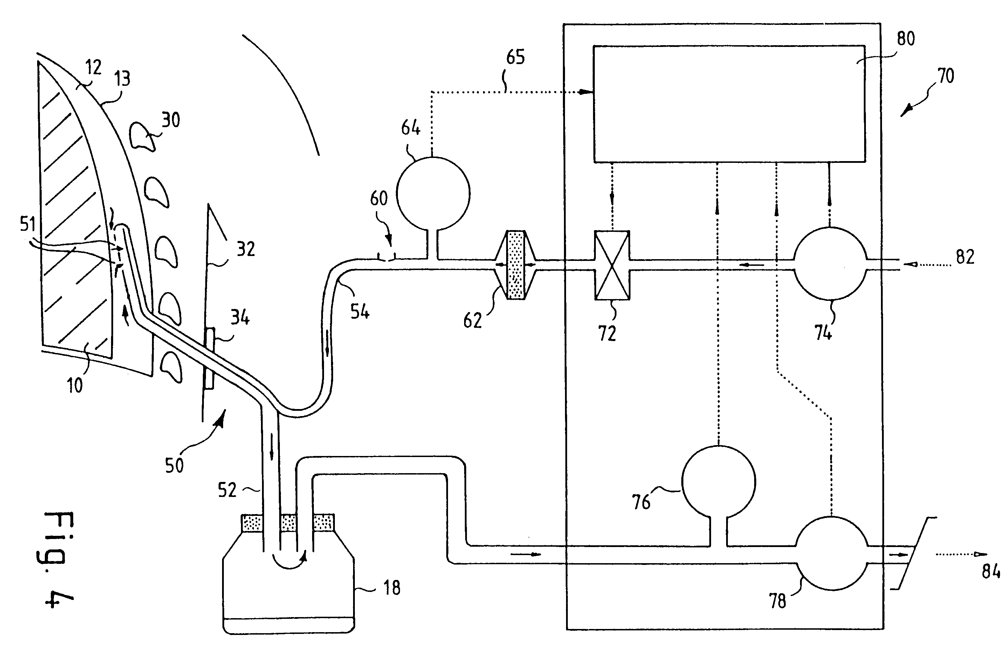

In Figur 4 ist eine bevorzugte Ausführungsform der erfindungsgemäßen Pleuradrainagevorrichtung dargestellt. In der zuführenden Zusatzleitung 54 ist, von der Patientenseite her gesehen, zunächst ein Zuspritzventil 60 und dann (stromaufwärts) ein Manometer 64 und ein Bakterienfilter 62 angeordnet. Das Manometer 64 kann auch stromaufwärts von dem Bakterienfilter 62 angeordnet sein. Innerhalb der Umrandung (Kasten 70) befindet sich der wiederverwendbare Teil der Vorrichtung mit dem Meß- und Steuergerät. Der wiederverwendbare Teil 70 weist zwei Durchflußmeßfühler 74 und 78 im zuführenden Teil 54 bzw. abführenden Teil 52 des Drains 50, ein Sicherheits-Sperrventil 72 und optional ein Manometer 76 auf. Das Manometer 76 ist nicht zwingend notwendig, da es an sich auch in üblichen Unterdruckquellen vorhanden ist. Ferner ist ein elektronisches Meß-, Steuer- und Anzeigegerät 80 vorgesehen. Wie durch den gestrichelten Pfeil 65 angedeutet, kann das Manometer 64 schaltungstechnisch mit dem Meß-, Steuer- und Anzeigegerät 80 verbunden sein oder selbst ein solches enthalten. Das Gerät 80 zeigt die Meßwerte und die sich aus ihnen ergebenden Rechengrößen, wie die Strömungsdifferenz zwischen Ab- und Zuleitung an, schließt bei Strömungsdifferenzumkehr, d.h. Einstrom größer als Ausstrom, das Sicherheits-Sperrventil 72 und sorgt bei intermittierendem Betrieb für den periodischen Verschluß des Ventils 72. Ferner zeigt es Störungen an und signalisiert Alarmzustände. Mit 82 ist der Luft- bzw. Spülgaseintritt der Zusatzleitung 54 bezeichnet, während der gestrichelte Pfeil am stromabwärtigen Ende der abführenden Leitung 52 zu einer Unterdruckquelle 84 führt.

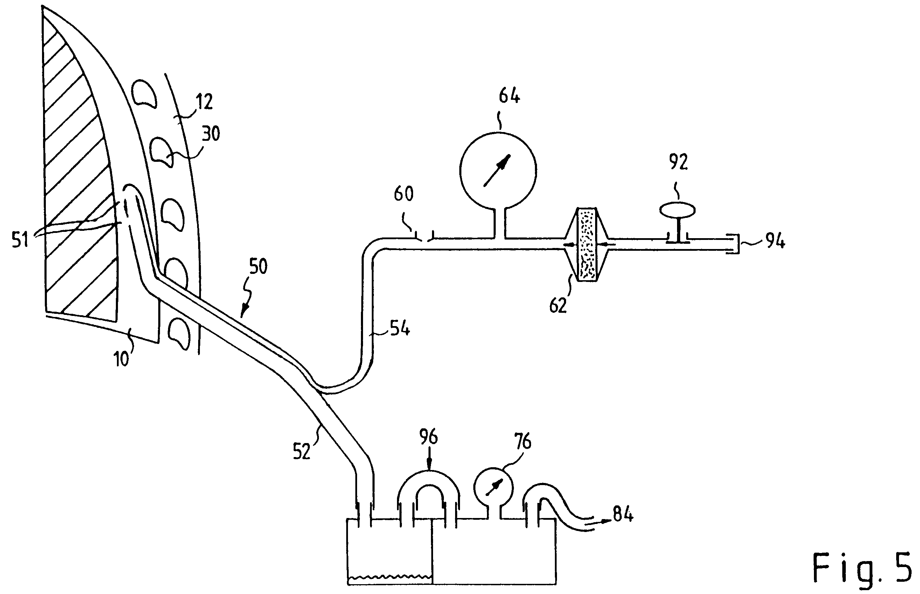

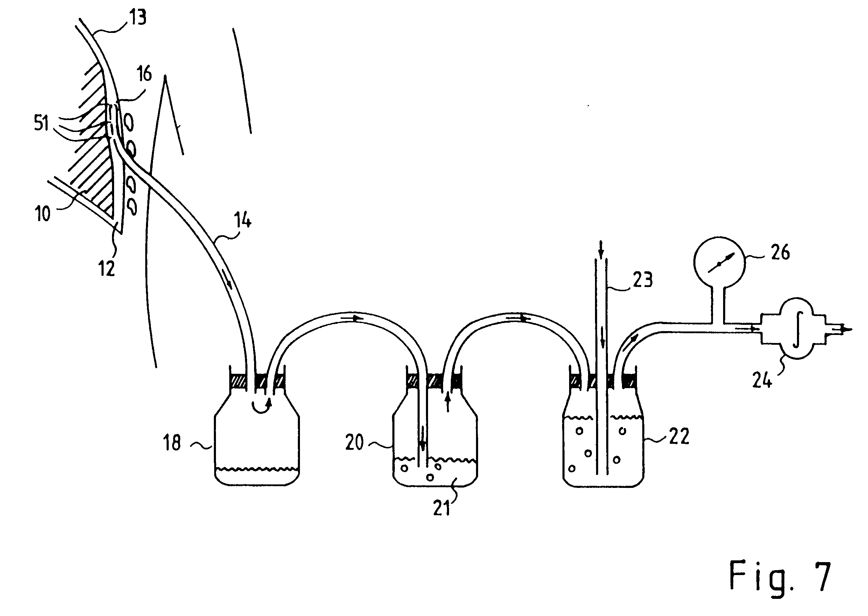

Bei der Ausführung gemäß Figur 5, die zu einer Zuschaltung der Zusatzleitung und der Meß- und Steuergeräte geeignet ist, ist im zuführenden Schenkel (Zusatzleitung) 54 ein Manometer 64 vorgesehen, das zwischen dem Zuspritzventil 60 und dem Bakterienfilter 62 liegt, sowie stromaufwärts ein Belüftungsventil 92 und ein abnehmbarer Verschluß 94. Statt des Verschlusses 94 kann auch ein Sperrventil zur programmierten intermittierenden Gasdurchspülung aufgesetzt sein, wie vorstehend beschrieben. Bei der Erweiterung ist ferner neben dem Manometer 76 bei 96 ein Durchflußmeßgerät (Flowmeter) angeschlossen, ebenso ist auch zwischen dem Belüftungsventil 92 und dem Verschluß 94 bzw. dem Sperrventil ein Durchflußmeßgerät (nicht dargestellt) geschaltet.

In der erfindungsgemäßen Pleuradrainagevorrichtung, vorzugsweise in der Zuführleitung 54, kann eine Auftrennungsmöglichkeit, beispielsweise in Form eines Kopplungsstücks vorgesehen sein, um einen Manipulatordraht einzuführen. Vorzugsweise ist die Auftrennungsmöglichkeit bzw. das Kopplungsstück (nicht dargestellt) zwischen dem Zuspritzventil 60 und dem Druckmeßgerät 64 in Fig. 4 bzw. Fig. 5 vorgesehen. Der Manipulatordraht 100 ist in Fig. 8 dargestellt und weist vorzugsweise eine Spitze 101, ein vorderes gerades Teil 102, eine Biegung oder Krümmung 103, ein hinteres gerades Teil 104 und ein gebogenes Ende 105 zur Erleichterung der Handhabung auf. Der Manipulatordraht besteht vorzugsweise aus einem leicht biegbaren Material, beispielsweise Aluminium oder Stahl mit Gummiummantelung, und ist in kliniküblicher Weise sterilisierbar. Mit Hilfe des Manipulatordrahtes läßt sich die Drainagesonde in ihrer Lage in der Pleurahöhle des Patienten abändern. Beispielsweise kann die Drainagesonde oder das Drain, wenn es dorsal liegt, und dadurch ventrale Luftansammlungen nur ungenügend abgesaugt werden können, nach ventral geschwenkt werden. Der Führungsstab bzw. Manipulatordraht wird dafür in die dem durchführenden Arzt geeignet erscheinende Form gebogen und mittels des Kopplungsstückes in die Zuführleitung 54 geschoben. Danach kann der Arzt versuchen, durch drehende und schwenkende Bewegungen die gewünschte Lageveränderung der Drainagesonde zur Verbesserung der Absaugung herbeizuführen. Danach wird der Draht abgezogen und die Drainage wieder in der vorher beschriebenen Weise angeschlossen und betrieben. Die Lageveränderung kann röntgenologisch kontrolliert werden. Ebenso ist es möglich, die Lageveränderung der Drainagesonde mittels des Manipulatordrahts unter unmittelbarer Röntgenkontrolle durchzuführen. Mittels des Meß- und Steuergeräts der erfindungsgemäßen Drainagevorrichtung kann ermittelt und angezeigt werden, ob der durch die Lageveränderung beabsichtigte Zweck, nämlich die Drainage zusätzlicher Gas- und Flüssigkeitsansammlungen, erreicht worden ist. Gegebenenfalls kann der Mainpulatordraht erneut in die Zuführleitung eingeführt und die Lage der Drainagesonde verbessert werden.

## Patentansprüche

1. Pleuradrainagevorrichtung zum Absaugen von Fluiden aus der Pleurahöhle, mit einem Drainageschlauch (52) zum Absaugen der Fluide, einer mit dem Drainageschlauch (52) in Verbindung stehenden Einrichtung (84) zum Ausbilden eines Unterdrucks in der Pleurahöhle (12), einer Zusatzleitung (54) zum Zuführen eines Gases, deren Lumen am patientenseitigen Ende in Fluidverbindung mit dem Lumen des Drainageschlauches (52) steht, wobei der Drainageschlauch (52) und die Zusatzleitung (54) zumindest teilweise als doppelläufiger Schlauch oder doppelläufige Drainagesonde (50) ausgebildet sind, und mit einer Einrichtung (70) zum Bestimmen der Differenz der Fluidströmungen in dem Drainageschlauch (52) und in der Zusatzleitung (54).

2. Vorrichtung nach Anspruch 1, wobei in der Zusatzleitung (54) ein Bakterienfilter (62) und/oder ein Sperrventil (72) und/oder ein Druckmeßgerät (64) und/oder ein Zuspritzventil (60) angeordnet ist.

3. Vorrichtung nach Anspruch 1 oder 2, wobei in der Zusatzleitung (54) und/oder im Drainageschlauch (52) ein Durchflußmesser (74) angeordnet ist.

4. Vorrichtung nach einem der Ansprüche 1 bis 3, wobei ferner eine Einrichtung (72) zum automatischen Sperren der Zusatzleitung (54) vorgesehen ist, wenn die Strömung in der Zusatzleitung (54) hoher ist als im Drainageschlauch (52).

5. Vorrichtung nach einem der Ansprüche 1 bis 4, wobei die Zusatzleitung (54) verschließbar und/oder wahlweise zuschaltbar ist.

6. Vorrichtung nach einem der Ansprüche 2 bis 5, wobei in der Zusatzleitung (54) vom patientenseitigen Ende her nacheinander das Zuspritzventil (60), das Druckmeßgerät (64), das Bakterienfilter (62) und ein Belüftungsventil (92) angeordnet sind.

7. Vorrichtung nach einem der Ansprüche 1 bis 6, wobei der Leitungsquerschnitt des Drainageschlauchs (52) größer ist als der Leitungsquerschnitt der Zusatzleitung (54).

8. Vorrichtung nach einem der Ansprüche 1 bis 7, wobei der Drainageschlauch (52) und die Zusatzleitung (54) in dem doppelläufigen Drain (50) koaxial ausgebildet sind oder parallel zueinander verlaufen.

9. Vorrichtung nach einem der Ansprüche 1 bis 8, wobei in der Zusatzleitung (54) eine Einrichtung zum Auftrennen der Zusatzleitung (54) und zum Einführen eines Manipulatordrahtes (100) vorgesehen ist.

## Claims

1. A pleura drainage apparatus for removing fluids from the pleural cavity by suction, comprising a drainage tube (52) for the suction-removal of the fluids, a means (84) communicating with the drainage tube (52) for generating a negative pressure in the pleural cavity (12), an additional conduit (54) for supplying a gas, whose lumen is in fluid communication with the lumen of the drainage tube (52) at the proximal end thereof, wherein the drainage tube (52) and the additional conduit (54) at least partially are provided as a double lumen tube or double lumen drainage probe (50), and comprising a means (70) for determining the difference of the fluid flows in the drainage tube (52) and in the additional conduit (54).

2. The apparatus according to claim 1, wherein a bacteria filter (62) and/or a stop valve (72) and/or a manometer (64) and/or an injection valve (60) is arranged in the additional conduit (54).

3. The apparatus according to claim 1 or 2, wherein a flow meter (74) is arranged in the additional conduit (54) and/or in the drainage tube (52).

4. The apparatus according to any one of claims 1 to 3, additionally comprising a means (72) for automatically blocking the additional conduit (54) if the flow in the additional conduit (54) exceeds that in the drainage tube (52).

5. The apparatus according to any one of claims 1 to 4, wherein the additional conduit (54) is closable and/or optionally connectable.

6. The apparatus according to any one of claims 2 to 5, wherein the injection valve (60), the manometer (64), the bacteria filter (62) and a venting valve (92) are consecutively arranged in the additional conduit (54) starting from the proximal end.

7. The apparatus according to any one of claims 1 to 6, wherein the cross-section of the drainage tube (52) is larger than the cross-section of the additional conduit (54).

8. The apparatus according to any one of claims 1 to 7, wherein the drainage tube (52) and the additional conduit (54) are arranged coaxially in the double lumen drain (50) or run in parallel to each other.

9. The apparatus according to any one of claims 1 to 8, wherein a means for separating the additional conduit (54) and for introducing a manipulator wire (100) is provided in the additional conduit (54).

## Revendications

1. Dispositif de drainage pleural pour aspirer des liquides hors de la cavité pleurale, avec un tube de drainage (52) pour aspirer les liquides, un dispositif (84) en liaison avec le tube de drainage (52) pour la formation d'une dépression dans la cavité pleurale (12), une canalisation supplémentaire (54) pour l'introduction d'un gaz, et dont la lumière est à l'extrémité du côté du malade en liaison fluide avec la lumière du tube de drainage (52), le tube de drainage (52) et la canalisation supplémentaire (54) étant au moins en partie sous la forme d'un tube double ou d'une sonde de drainage double (50), et avec un dispositif (70) pour déterminer la différence entre les courants de fluide dans le tube de drainage (52) et la canalisation supplémentaire (54).

2. Dispositif selon la revendication 1, dans lequel est disposé(e) dans la canalisation supplémentaire (54) un filtre à bactéries (62) et/ou une soupape de fermeture (72) et/ou un appareil de mesure de la pression (64) et/ou une soupape d'arrosage (60).

3. Dispositif selon la revendication 1 ou 2, dans lequel est disposé dans la canalisation supplémentaire (54) et/ou dans le tube de drainage (52) un appareil de mesure du débit (74).

4. Dispositif selon l'une des revendications 1 à 3, dans lequel en outre est prévu un dispositif (72) pour la fermeture automatique de la canalisation supplémentaire (54) lorsque le débit dans la canalisation supplémentaire (54) est supérieur à celui du tube de drainage (52).

5. Dispositif selon l'une des revendications 1 à 4, dans lequel la canalisation supplémentaire (54) peut être fermée et/ou connectée selon les besoins.

6. Dispositif selon l'une des revendications 2 à 5, dans lequel dans la canalisation supplémentaire (54) sont disposé(e)s successivement, en partant du côté du malade, la soupape d'arrosage (60), l'appareil de mesure de la pression (64), le filtre à bactéries (62) et une soupape d'aération (92).

7. Dispositif selon l'une des revendications 1 à 6, dans lequel le diamètre de canalisation du tube de drainage (52) est supérieur à celui de la canalisation supplémentaire (54).

8. Dispositif selon l'une des revendications 1 à 7, dans lequel le tube de drainage (52) et la canalisation supplémentaire (54) dans le drain double (50) sont disposés de façon coaxiale ou courent parallèlement entre eux.

9. Dispositif selon l'une des revendications 1 à 8, dans lequel dans la canalisation supplémentaire (54) est prévu un appareil pour la diviser et pour introduire un fil manipulateur (100).
